# EUROPEAN PATENT APPLICATION

(11) **EP 4 582 561 A1**
(43) Date of publication of application: **09.07.2025**
(21) Application number: 22956991.8
(22) Date of filing: 02.09.2022
(51) Int. Cl.: C12Q 1/6886, G01N 33/574

(54) **COMBINATION OF DIFFERENTIALLY METHYLATED REGIONS, KIT, AND USE THEREOF**

(71) Applicant: BGI Genomics Co., Limited, Guangdong 518083 (CN)
(72) Inventor: LI, Zhilong, Shenzhen, Guangdong 518083 (CN); WANG, Yuying, Shenzhen, Guangdong 518083 (CN); PENG, Jiaxi, Shenzhen, Guangdong 518083 (CN); JIANG, Ruijingfang, Shenzhen, Guangdong 518083 (CN); SUN, Jianlong, Shenzhen, Guangdong 518083 (CN)
(74) Representative: Inspicos P/S
(86) International application number: PCT/CN2022/116795
(87) International publication number: WO 2024/045170

(57) **Abstract**

Provided are a combination of differentially methylated regions, a kit, and use thereof. The combination of differentially methylated regions comprises the following differentially methylated regions: (1) chr8: 145106171-145107467 and (2) chr8:67344198-67345563. The combination of differentially methylated regions can be used for effective detection of colorectal cancer or precancerous lesions of colorectal cancer, or for evaluating prognostic risks of colorectal cancer patients, exhibiting high sensitivity and specificity.

## Description

### Technical Field

The present invention belongs to the field of biomedicine, and relates to a combination of differentially methylated regions of OPLAH gene and ADHFE1 gene, a kit and use thereof. Specifically, the kit is a kit for detecting the differentially methylated region combination. Specifically, the use is for detecting colorectal cancer, or for assessing the prognostic risk of colorectal cancer patients.

### Background Art

Colorectal cancer occurring in the colon and rectum of the lower digestive tract is a common malignant tumor. According to the latest statistics, the number of new cases of colorectal cancer ranks third among men and second among women worldwide, and the number of deaths ranks fourth among men and third among women (Bray et al., 2018). In China, the prevention and control situation of colorectal cancer is also very severe, which ranks third among new cases of malignant tumors (388,000) and fifth among cases of deaths (187,000) (Zheng Rongshou et al., 2019). Colorectal cancer develops slowly, generally going through polyps, adenomas, and intestinal cancer, and it can take up to 5 to 10 years for adenoma to develop into intestinal cancer. If intervention is carried out in the early stages of colorectal cancer, the mortality rate can be significantly reduced. The five-year survival rate of patients with stage I colorectal cancer can reach more than 90%, while the five-year survival rate of patients with stage IV colorectal cancer is less than 20% (Marzieh Araghi et al., 2020).

Traditional colorectal cancer early screening technologies mainly include colonoscopy, fecal occult blood detection, tumor markers CEA and CA19-9, etc. These technical means currently have certain limitations. Colonoscopy is the gold standard for colorectal cancer diagnosis. Lens and light source can be inserted from anus, move along cavity to pass through rectum, sigmoid colon and other parts to reach the end of ileum, and images can be transmitted to display in real time for the operating doctor to observe. The images of colonoscope are intuitive and clear, and various lesions such as cancer, polyps, ulcers, and bleeding can be observed. Polyps can also be removed under microscope. However, it is an invasive detection technology with a complicated preparation process, requiring diet control and intestinal cleaning, and the compliance of examinees is low. In addition, it also requires equipment and personnel, and needs to be operated by hospital professionals and anesthesiologists. Some examinees will feel uncomfortable and have the risk of complications (3 to 5 cases/1000 people). Fecal occult blood detection is a non-invasive method that detects blood components (hemoglobin) in stool to determine whether there is gastrointestinal bleeding, so that doctors can judge the risk of colorectal cancer. This method is quick and simple, and the compliance of examinees is high. However, this detection method is easily affected by liver, blood products, green leafy vegetables, etc. in the diet, resulting in false positives. At the same time, it has low sensitivity for early colorectal cancer patients with less bleeding, and is prone to missed diagnosis. CEA and other broad-spectrum tumor markers have low specificity, are prone to more false positives, and have limited sensitivity. Therefore, it is in need to establish an accurate, simple and economical early screening method for colorectal cancer.

DNA methylation is an important gene expression regulation mechanism that can regulate gene expression and silencing, and has a significant impact on the occurrence and development of tumors. Abnormal methylation of cancer-related genes often occurs in the early stages of cancer, so DNA methylation signals are considered to be potential early screening markers for tumors. Human fecal samples are mixed with intestinal cell DNA, which carries information such as methylation. By detecting and analyzing this information, the possibility of examinee suffering from colorectal cancer can be inferred. Some genes have been proven to be methylation markers for colorectal cancer detection in feces, such as SFRP2, SEPT9, NDRG4 and SDC2 (Hannes M Müller et al., 2004; Jie Chen et al., 2019), but their performance cannot fully meet clinical needs. For example, the sensitivity and specificity of SFRP2 for colorectal cancer are only 77% (Hannes M Müller et al., 2004).

OPLAH gene encodes 5-hydroxyproline enzyme and affects the glutamate salvage pathway and abnormal expression. Studies have shown that the downregulation of expression caused by abnormal hypermethylation of OPLAH affects the metabolism of glutathione and is accompanied by tumorigenesis (Liu Y et al., 2014).

ADHFE1 encodes a transhydrogenase, and abnormal regulation caused by hypermethylation of this gene can shorten the cycle of colorectal cancer cells and promote cancer cell proliferation (Tae CH, Ryu KJ, Kim SH, Kim HC, Chun HK, Min BH, et al. Alcohol dehydrogenase, iron containing, 1 promoter hypermethylation associated with colorectal cancer differentiation. BMC Cancer. 2013;13:142.).

At present, there is still a need to develop new markers for colorectal cancer detection.

### Contents of the present invention

After in-depth research and creative work, the inventors discovered the differentially methylated region (DMR) of the OPLAH gene and the differentially methylated region of the ADHFE1 gene. The inventors surprisingly found that the combination of the differentially methylated regions can be used to effectively detect colorectal cancer or colorectal cancer precancerous lesions, or to assess the prognostic risk of colorectal cancer patients, with good sensitivity and specificity. The following invention is thus provided:
One aspect of the present invention relates to a differentially methylated region, which comprises the following differentially methylated regions:
(1) chr8: 145106171-145107467 (DMR1), and
(2) chr8: 67344198-67345563 (DMR2).

The (1) above is a differentially methylated region of OPLAH gene.

The (2) above is a differentially methylated region of ADHFE1 gene.

The (1) and (2) above can also be referred to as a combination of differentially methylated regions.

In some embodiments of the present invention, the differentially methylated region consists of differentially methylated regions (1) and (2).

In some embodiments of the present invention, in the differentially methylated region:
the differentially methylated region shown in item (1) (DMR1) has a base sequence as set forth in SEQ ID NO: 1 or a complementary sequence thereof, and/or
the differentially methylated region shown in item (2) (DMR2) has a base sequence as set forth in SEQ ID NO: 2 or a complementary sequence thereof.

The base sequence of DMR1 is:

CGCCGTAGCTGCGTCCCCAGAACCGGGAGACTTAAGGCATCTTTATTGCGGGAT
CCTCACACGGCCTCCTGGGCCCGGCGATACTCATAGACGCTGCCGTGCTCGGGAAA
GGCCAGTGCTTGCGGGGGCGACCCCGGCGGTGGGGCGGGGTCCTCCGGGTCCCCAT
AGCCACCGCCGCCGGGCGTGTGGAGACAGAACACATCCTGTTGGCGCGGGGGGGGG
CGGGGAGGCGGGCTCAGTGCAGGCGTGGCCCCTCCCTGTCACCTGCGCAGTCCCCTC
CCCACCGGGCAGCGTCCATCACCCGCCCCCATGGACCACGGAGGCCCGGTACGCGC
CCCCTTTCCTTCCGGGGCCAAATTAAACTCGGCCTCTGGTCAGGTCTGCAACTGTTCC
CCCTCTGTGGGATGGGGCCGGGAGGGGAGGGGAGGGGAGGGGGACAGGACAGGCC

GCGGCCCTTACCCCGGGGTACACGGTCACCGACGTCTTGCCGCCCAGATTCACCGTC
CGGCCGTTTTTGCGGATCAGCAGGTTTAGGCCGCGGGCGCCAGGCTCGCCCCCTGCG
GAGGGAGGCGAGGAGTCCAGAGAGACCAGGGGCGGGGGTGGGGGCGGGGGCGGG
GAGGGCCGCGAGGGCTGGGGAACCGCGCACCGTGGAGCCCGTATGGCCGGAAGGC
GCGGCGCTCGGTCAGCACTGACAGCAGCGCCTCCTCACGAAAGAGCAGCTCGCGGG
TGACGCCGTCGCCGCCTCGGAAGCGGCCTCTGCCCCCCGAGCCCCCCGCCGCAGCTC
GAAGCGGCGCAGGATGACCGGGTACCTGCGAGGGCGAGGACGAGGGCAAAGACTC
AGCGTGGCCCGAGCCCCGGCTCCCACCCGGCCGTGCCCCCAGCCTCCGCGCACGCC
GCTCACCGGCTCTCCAGGATCTCAGGGTCGGTGATGCGTGTGTTGGTCATGTGGCTG
TGCACACCGCTGCGCCCGTGCCAGCTGGGACCCGCGCCCGCGCCGCCCGCCACCGTC
TCGTAGTAGCCCATGTGGGCGTTGCCCAGGGTCACGTTGTTCATGCAGCCCTGGTGA
GGGCACCTGGTCGCTGCGCTGGGCTGGGGCAGGGCGTCCCCACCTCGCCCTCCCGCT
CCTACTCCAGCCTGCCCACCCAGTCCGCCCCCGAGCTGCGCCCACCCCGCCCCCGCA
CCTGGGAGGCGGCGCAGGCCCCAAAGGCCCCCAGGATGACATCCACCACGCGCTGC
GACGTGAGCACGTTGCCGCCCACCACCGCCGCCTCGGGCGACGGGTCCAGGATGGA
GCCTC (SEQ ID NO: 1)

The base sequence of DMR2 is:

AGTCTAATCATGGCAAGTTTTAACTATTGATGTAAGAGTTTTAACTATTGATTTT
GCTTTCATCAAAAGCAAAGCCATTCACACAGTATTTAACAAAAACCCTCGGGCTCTG
CTAGGTTCTTGTTTACTGTTAAAACAGTTTAAAGGCAGGGTCTTGATCACTTAAAAT
ACTCTTAGAAGGGAAAGTAACATTTTAAAGGATTAACAGTGGAAAAAGAATATCCC
TGTTCCTCTTCCTTCCCCCAAGTCCTCAACATGTGAAAAATAGAGTTGATAAGTAAG
GAGATTTAAGGCAGAACCCGAGGCCTACGGAGCAGTTACCTTCTACGGCAATTTCA
AGGGTGGATGGTGCGAGCGCCGCTGGGGCAGCTGGCGTTCTGGTTCTTACTCCGTGG
GAAAATGGCCCTGAGCCCGACTGGCTTGAGGCTTAGACAGGTGACCCCGCGAAGCG
GGTGGGCAGGCGCGGCCGAGGGGCGGGAGGCGGGCAGCCTCCGTGATTGGCCGGC
GGAGGGGGCGGTGTAGCGACCCGAGGAGGGAAGAGGACTCCAAGCGCCATGGCCG
CTGCCGCCCGAGCCCGGGTCGCGTACTTGCTGAGGCAACTGCAACGCGCAGCGTGA
GTGCGGGGCCGGCGGGCGGGCAGGGGACCGCAGGGTGCCCACGCAGCCCCCTGGGT
GTGACCCGCTCAGATCCTGCGCAGTCTTCTCCGCTTACTTTCAAGAATTCGGAGAAT
TTGGATACCGCCCTGGGCTCCCACCGCGCGCCAGGCGGAACAGCGAGGTCACAGAG
TGCGCGATGCAGCCTCTCAGGGCGCAGTGAGGCAGCCTTTACTCTGCTTGTCTACCG
TTAAAGAAACTGAGGCCCAGAGAGGACCTGGTCCCACATCGTAGCAGGCAGTGGAG
AGCTTGGCACGGTGGCCAAGACAAATTCATGTATGCAATTGACACATATTTATTGGG
CATATATTATGTGCCAAAAACTATTCTAGGTTCTGGGGATACAAAGATGAAAGGCTC

TGGAGCAGTTTGAGGTCTAGTGGGGAATGGGATGAGGGGACGAGGCTAAAACCGGC
TATAGCAGACTGGGTGTAGAAGGGAAGGATGGATGTGAGAAGTCGCAGAGTCACAC
TTCCTGGCATGTACAGGGAGTCAGGGTATTCAATGTCATGACACGCCTGCCTCCCAG
CAGAAATGTGAGGTACATCCTGGTCACCTGATAAATACTTGCTGACTGTGCAAGTGC
ACAAGTCTGCCTGGGAAACATGGAAAAGTTTGAGCTCACAGGTGCAGATCAGTAGG
AGGAAAGGCGAGGGACTGGCGAGGAACGTCAGGCAGAGAGAAGAGCAACACCAGC
GATGGAGATGCAGTCC (SEQ ID NO:2)

In some embodiments of the present invention, the differentially methylated region is used to detect a colorectal cancer or a colorectal cancer precancerous lesion, or to assess the prognostic risk of a colorectal cancer patient.

In some embodiments of the present invention, the differentially methylated region has undergone the following treatment:
a bisulfite treatment, a methylation sensitive endonuclease treatment, a methylation restriction endonuclease treatment or a methylation modification enzyme treatment.

The differentially methylated region of the present invention is an OPLAH gene methylation marker or an ADHFE1 gene methylation marker for colorectal cancer detection. The combination of differentially methylated regions is a combination of an OPLAH gene methylation marker and an ADHFE1 gene methylation marker for colorectal cancer detection.

Another aspect of the present invention relates to a kit, comprising a reagent for detecting the differentially methylated region described in any one of items of the present invention or a reagent for detecting a methylation site in the differentially methylated region.

In some embodiments of the present invention, in the kit, the reagent comprises:
1) a primer and a probe for detecting the differentially methylated region shown in item (1) or a methylation site within the differentially methylated region;
2) a primer and a probe for detecting the differentially methylated region shown in item (2) or a methylation site within the differentially methylated region;
preferably, the reagent also comprises:
3) a primer and a probe for an internal reference gene.

In some embodiments of the present invention, in the kit:
in 1), the sequence of the primer is selected from SEQ ID NOs: 3 to 79, and the sequence of the probe is selected from SEQ ID NOs: 160 to 188; and in 2), the sequence of the primer is selected from SEQ ID NOs: 80 to 159, and the sequence of the probe is selected from SEQ ID NOs: 189 to 209;
preferably:
   in 1), the sequence of the primer is selected from SEQ ID NO: 30 and SEQ ID NO: 32, and the sequence of the probe is selected from SEQ ID NO: 170; and in 2), the sequence of the primer is selected from SEQ ID NOs: 108 to 109, and the sequence of the probe is selected from SEQ ID NO: 197;
   in 1), the sequence of the primer is selected from SEQ ID NOs: 25 to 26, and the sequence of the probe is selected from SEQ ID NO: 164; and in 2), the sequence of the primer is selected from SEQ ID NOs: 144 to 145, and the sequence of the probe is selected from SEQ ID NO: 204;
   in 1), the sequence of the primer is selected from SEQ ID NOs: 26 to 27, the sequence of the probe is selected from SEQ ID NO: 168; and in 2), the sequence of the primer is selected from SEQ ID NOs: 154 to 155, the sequence of the probe is selected from SEQ ID NO: 206;
   in 1), the sequence of the primer is selected from SEQ ID NOs: 33 to 34, the sequence of the probe is selected from SEQ ID NO: 171; and in 2), the sequence of the primer is selected from SEQ ID NOs: 156 to 157, the sequence of the probe is selected from SEQ ID NO: 209;
   in 1), the sequence of the primer is selected from SEQ ID NOs: 56 to 57, the sequence of the probe is selected from SEQ ID NO: 184; and in 2), the sequence of the primer is selected from SEQ ID NOs: 158 to 159, the sequence of the probe is selected from SEQ ID NO: 203;
   in 1), the sequence of the primer is selected from SEQ ID NOs: 61 to 62, the sequence of the probe is selected from SEQ ID NO: 183; and in 2), the sequence of the primer is selected from SEQ ID NOs: 108 to 109, the sequence of the probe is selected from SEQ ID NO: 191;
   in 1), the sequence of the primer is selected from SEQ ID NOs: 33 to 34, the sequence of the probe is selected from SEQ ID NO: 171; and in 2), the sequence of the primer is selected from SEQ ID NOs: 108 to 109, the sequence of the probe is selected from SEQ ID NO: 191;
      or,
   in 1), the sequence of the primer is selected from SEQ ID NOs: 25 to 26, the sequence of the probe is selected from SEQ ID NO: 164; and in 2), the sequence of the primer is selected from SEQ ID NOs: 158 to 159, the sequence of the probe is selected from SEQ ID NO: 203;
   preferably, in 3), the sequence of the primer of the internal reference gene is set forth in SEQ ID NOs: 210 to 211, and the sequence of the probe of the internal reference gene is set forth in SEQ ID NO: 212.

In some embodiments of the present invention, the kit further comprises one or more selected from the following:
a stool sample sampler, a nucleic acid extraction reagent, a methylation detection sample pretreatment reagent, a PCR reagent, and a stool occult blood detection reagent;
preferably, the stool occult blood detection reagent is a stool immunochemical detection reagent, a guaiac detection reagent, a tetramethylbenzidine detection reagent, or an ELISA double antibody sandwich detection reagent;
preferably, the stool immunochemical detection reagent is an immunocolloidal gold fecal occult blood detection reagent.

In some embodiments of the present invention, in the kit, the methylation detection sample pretreatment reagent is a bisulfite, a methylation sensitive endonuclease, a methylation restriction endonuclease, or a methylation modifying enzyme.

Another aspect of the present invention relates to the use of the differentially methylated regions of the present invention, or a reagent for detecting the differentially methylated regions, or a methylation site within any one of the differentially methylated regions of the present invention in the manufacture of a medicament for detecting a colorectal cancer or a colorectal cancer precancerous lesion, or for assessing the prognostic risk of a colorectal cancer patient.

In some embodiments of the present invention, in the use, the reagent comprises a primer and a probe for detecting the differentially methylated regions as described in any one of items of the present invention or a methylation site in the differentially methylated regions.

Another aspect of the present invention relates to a method for detecting a colorectal cancer or a colorectal cancer precancerous lesion, or a method for assessing the prognostic risk of a colorectal cancer patient, comprising the following steps:
detecting the content of the differentially methylated region described in any one of items of the present invention or the content of a fragment thereof; wherein the fragment comprises one or more methylation sites in the differentially methylated region.

Those skilled in the art can understand that the detected content of a fragment in the differentially methylated region, such as the content of the fragment detected by a PCR method, can represent the content of the differentially methylated region.

In some embodiments of the present invention, in the method, the content of each differentially methylated region or a fragment thereof as described in any one of items of the present invention is detected by a fluorescent quantitative PCR method.

In some embodiments of the present invention, in the method,
the content of human DNA in the sample is evaluated by an internal reference gene Ct value, when the internal reference gene Ct value is >37, the sample is judged to have too low human DNA content and the detection fails; when the internal reference gene Ct value is ≤37, the sample is evaluated to be qualified and can be analyzed later.

In some embodiments of the present invention, in the method, for the sample that is evaluated to be qualified:
when the Ct value of each differentially methylated region is greater than 38 or no Ct value is detected, it is judged to have a negative detection result;
when the Ct value of each differentially methylated region is less than or equal to 38, the difference △Ct value between each differentially methylated region and the internal reference gene Ct value is further calculated.

In some embodiments of the present invention, in the method:
if the △Ct of item (1) or item (2) ≤ a cutoff value, it is judged to have a high risk of colorectal cancer, otherwise it is judged to have a low risk;
wherein △Ct = (Ct value of the differentially methylated region or Ct value of the amplified fragment) - Ct value of the reference gene;
wherein, the amplified fragment comprises one or more methylation sites in the differentially methylated region.

In some embodiments of the present invention, in the method:
the cutoff value of item (1) is 7, and/or
the cutoff value of item (2) is 12.

In some embodiments of the present invention, the method further comprises a step of performing an evaluation in combination with a fecal occult blood detection result.

In some embodiments of the present invention, in the method, the sample is a human tissue sample, a blood sample, a cell sample, a secretion sample or an excrement sample such as a fecal sample.

According to some embodiments of the present invention, a fecal sample is used as a test object. In this way, the fecal sample comprises intestinal exfoliated cells, meets the requirements of the test sample, is non-invasive, and is more patient-friendly.

According to some embodiments of the present invention, the optional detection means include but are not limited to: Sanger sequencing, pyrophosphate sequencing, high-throughput sequencing, nucleic acid mass spectrometry, PCR, fluorescence quantitative PCR, ddPCR, etc. Since the nucleic acid sequence has not changed, methylation modification is difficult to be directly detected. The nucleic acid often needs to be pretreated during the experiment. The pretreatment means herein include but are not limited to: a bisulfite treatment, a methylation sensitive endonuclease treatment, a methylation restriction endonuclease treatment or a methylation modification enzyme treatment. At the same time, the third-generation sequencing technology has been proven to be used to directly determine the methylation modification state of DNA, and with the development of technology, it can also be used as a detection means in the future. Fluorescence quantitative PCR technology is one of the commonly used and mature detection technologies for molecular diagnosis, which is fast, accurate and low-cost. This technical solution adopts a method of combining bisulfite treatment with fluorescence quantitative PCR, and designs several methylation-specific PCR primers and probes for the OPLAH gene and ADHFE1 gene, which can specifically identify methylated DNA molecules, realize the detection of the methylation state of the sample, and then evaluate the cancer risk of the subject.

By performing whole genome methylation sequencing and targeted methylation sequencing on colorectal cancer tissue and control tissue samples, methylation differential regions highly associated with colorectal cancer were obtained, among which the methylation rates of DMR1 (chr8: 145106171-145107467) and DMR2 (chr8: 67344198-67345563) were particularly significant. Primers and probes were designed for the above regions, which could be used to quickly and accurately detect the methylation of OPLAH and ADHFE1 genes. The present invention provides several primer and probe sequences, as shown in Table 1:

**Table 1: Primer and probe sequences of OPLAH gene and ADHFE1 gene**

| SEQ ID No. | Sequence (5'-3') | Description |
|---|---|---|
| 3 | GAATTAGGCGTCGTAGTTGC | OPLAH gene primers |
| 4 | CGCAAACACTAACCTTTCCC | |
| 5 | GGGTTCGGCGATATTTATAGAC | OPLAH gene primers |
| 6 | CCCCACCGCCGAAATC | |
| 7 | AGTACGGTAGCGTTTATGAGTATC | OPLAH gene primers |
| 8 | AACTAAACGCCGTAACTACG | |
| 9 | TTATCGTCGGGGTCGTTTTC | OPLAH gene primers |
| 10 | ACATCTTTATTACGAAATCCTCACAC | |
| 11 | TCGTCGGGTTTAGGAGGTC | OPLAH gene primers |
| 12 | ACTCGAAACACGAACTAAACG | |
| 13 | TATTTTTATTGCGGGATTTTTATAC | OPLAH gene primers |
| 14 | CAAAATATATTCTATCTCCACACGC | |
| 15 | CGTAGTTGCGTTTTTAGAATCG | OPLAH gene primers |
| 16 | TATAAAAACCCGAAAAACCCCG | |
| 17 | GGGTAGTGCGTTTGTAGTTTC | OPLAH gene primers |
| 18 | CGACAACGTCTATAAATATCGC | |
| 19 | TTTTGGAGGGGTTTACGGATTATC | OPLAH gene primers |
| 20 | CCAAAAAACCGTATAAAAATCCCG | |
| 21 | AAGGTAAGTATAGATGAGGGGCG | OPLAH gene primers |
| 22 | CGCAAACACTAACCTTTCCCG | |
| 23 | GAGGTCGTGTGAGGATTTCG | OPLAH gene primers |
| 24 | AACAATACGCCTACAACTCCG | |
| 25 | GGGTAGTGCGTTTGTAGTTTCG | OPLAH gene primers |
| 26 | ACACGACAACGTCTATAAATATCGC | |
| 27 | TCGGGTAGTGCGTTTGTAGTTTC | OPLAH gene primers |
| 26 | ACACGACAACGTCTATAAATATCGC | |
| 28 | GGTTTACGGATTATCGGGTAGTGC | OPLAH gene primers |
| 29 | CGACAACGTCTATAAATATCGCCG | |
| 30 | GGGCGTTGTTTAGGGTTAC | OPLAH gene primers |
| 31 | AAACGCAACTCGAAAACG | |
| 30 | GGGCGTTGTTTAGGGTTAC | OPLAH gene primers |
| 32 | CCCTACCCCAACCCAAC | |
| 33 | CGTTCGTGTTAGTTGGGATTC | OPLAH gene primers |
| 34 | CATAAACAACGTAACCCTAAACAAC | |
| 35 | GGGACGTTTTGTTTTAGTTTAGC | OPLAH gene primers |
| 36 | CCCGTACCAACTAAAACCC | |
| 37 | TATTTGCGAGGGCGAGGAC | OPLAH gene primers |
| 38 | CCTAAAAAACCGATAAACGACG | |
| 39 | CGTTCGTGTTAGTTGGGATGC | OPLAH gene primers |
| 34 | CATAAACAACGTAACCCTAAACAAC | |
| 33 | CGTTCGTGTTAGTTGGGATTC | OPLAH gene primers |
| 40 | TAAACAACGTAACCCTAAACACCG | |
| 41 | GGGCGTTGTTTAGGGTTGC | OPLAH gene primers |
| 32 | CCCTACCCCAACCCAAC | |
| 30 | GGGCGTTGTTTAGGGTTAC | OPLAH gene primers |
| 42 | CCTACCCCAACCCACCG | |
| 43 | CGTTCGTGTTAGTTGGGCTTC | OPLAH gene primers |
| 34 | CATAAACAACGTAACCCTAAACAAC | |
| 33 | CGTTCGTGTTAGTTGGGATTC | OPLAH gene primers |
| 44 | TAAACAACGTAACCCTAAACCACG | |
| 45 | CGTTCGTGTTAGTTGGGATCC | OPLAH gene primers |
| 34 | CATAAACAACGTAACCCTAAACAAC | |
| 46 | CGTTCGTGTTAGTTGGGGTTC | OPLAH gene primers |
| 34 | CATAAACAACGTAACCCTAAACAAC | |
| 33 | CGTTCGTGTTAGTTGGGATTC | OPLAH gene primers |
| 47 | TAAACAACGTAACCCTAAACGACG | |
| 33 | CGTTCGTGTTAGTTGGGATTC | OPLAH gene primers |
| 48 | TAAACAACGTAACCCTAAACAACG | |
| 30 | GGGCGTTGTTTAGGGTTAC | OPLAH gene primers |
| 49 | CCTACCCCAACCCAACG | |
| 50 | AAGCGGCGTAGGATGATC | OPLAH gene primers |
| 51 | CCGAAACTCGAACCACG | |
| 50 | AAGCGGCGTAGGATGATC | OPLAH gene primers |
| 52 | AAACCGAAACTCGAACCAC | |
| 33 | CGTTCGTGTTAGTTGGGATTC | OPLAH gene primers |
| 53 | TTTAAAACCTACGCCGCCT | |
| 33 | CGTTCGTGTTAGTTGGGATTC | OPLAH gene primers |
| 54 | ACGAAATAAAAACGCCCTACC | |
| 33 | CGTTCGTGTTAGTTGGGATTC | OPLAH gene primers |
| 31 | AAACGCAACTCGAAAACG | |
| 33 | CGTTCGTGTTAGTTGGGATTC | OPLAH gene primers |
| 55 | ACTCGAAAACGAACTAAATAAACAAACT | |
| 56 | TGAATAACGTGATTTTGGGTAACG | OPLAH gene primers |
| 57 | CGACTCTCCAAAATCTCAAAATCG | |
| 58 | GGGTAAAGATTTAGCGTGGTTC | OPLAH gene primers |
| 48 | TAAACAACGTAACCCTAAACAACG | |
| 33 | CGTTCGTGTTAGTTGGGATTC | OPLAH gene primers |
| 32 | CCCTACCCCAACCCAAC | |
| 33 | CGTTCGTGTTAGTTGGGATTC | OPLAH gene primers |
| 59 | ACGAAATAAACGCAACTCG | |
| 33 | CGTTCGTGTTAGTTGGGATTC | OPLAH gene primers |
| 60 | CGCAACTCGAAAACGAACT | |
| 61 | AGGATGATCGGGTATTTGCG | OPLAH gene primers |
| 62 | CAACCACATAACCAACACACG | |
| 61 | AGGATGATCGGGTATTTGCG | OPLAH gene primers |
| 63 | GCGAATCCCAACTAACACG | |
| 64 | GACGAGGGTAAAGATTTAGCG | OPLAH gene primers |
| 63 | GCGAATCCCAACTAACACG | |
| 65 | GGTAAAGATTTAGCGTGGTTCG | OPLAH gene primers |
| 48 | TAAACAACGTAACCCTAAACAACG | |
| 66 | TCGGTTTTTTAGGATTTTAGGGTCG | OPLAH gene primers |
| 67 | CCTCACCAAAACTACATAAACAACG | |
| 68 | TTGTATGAATAACGTGATTTTGGGTAACG | OPLAH gene primers |
| 69 | CACCGACTCTCCAAAATCTCAAAATCG | |
| 70 | TTATCGATTTTGAGATTTTGGAGAGTCG | OPLAH gene primers |
| 71 | CTCGATCAACACTAACAACAACG | |
| 72 | GCGTAGGATGATCGGGTATTTGC | OPLAH gene primers |
| 73 | CGAATCCCAACTAACACGAACG | |
| 74 | TTTGCGGAGGGAGGCG | OPLAH gene primers |
| 75 | GCGATTCCCCAACCCTCG | |
| 76 | GGAGTTTAGAGAGATTAGGGGCG | OPLAH gene primers |
| 77 | CGACCATACGAACTCCACG | |
| 78 | TCGTGGAGTTCGTATGGTCG | OPLAH gene primers |
| 79 | CCCGCGAACTACTCTTTCG | |
| 80 | TCGTTGGGGTAGTTGGC | ADHFE1 gene primers |
| 81 | CCTATCTAAACCTCAAACCAATCG | |
| 82 | GTTTTGGGTTTTTATCGCGC | ADHFE1 gene primers |
| 83 | GCCCTAAAAAACTACATCGCG | |
| 84 | AGGTTTAGATAGGTGATTTCGC | ADHFE1 gene primers |
| 85 | AAACTACCCGCCTCCC | |
| 86 | TTGGCGTTTTGGTTTTTATTTC | ADHFE1 gene primers |
| 81 | CCTATCTAAACCTCAAACCAATCG | |
| 87 | GATTTAAGGTAGAATTCGAGGTTTAC | ADHFE1 gene primers |
| 88 | CACGAAATAAAAACCAAAACG | |
| 89 | CGTAGTTTTTTGGGTGTGATTC | ADHFE1 gene primers |
| 90 | AAAACGATATCCAAATTCTCCG | |
| 91 | CGTATTTTGTGATTTCGTTGTTTC | ADHFE1 gene primers |
| 92 | AAAAATTCGAAAAATTTAAATACCG | |
| 93 | CGTTTGTTTATTCGTTTCGC | ADHFE1 gene primers |
| 94 | CGCCGCTAAAACAACTAACG | |
| 95 | CGTTTTGAGAGGTTGTATCGC | ADHFE1 gene primers |
| 92 | AAAAATTCGAAAAATTTAAATACCG | |
| 96 | GTTTAGGGCGGTATTTAAATTTTTC | ADHFE1 gene primers |
| 97 | ATATAACCCGCTCAAATCCTACG | |
| 98 | TGTATCGCGTATTTTGTGATTTC | ADHFE1 gene primers |
| 99 | CCGCTTACTTTCAAAAATTCG | |
| 100 | GGTAATTTTAAGGGTGGATGGTG | ADHFE1 gene primers |
| 101 | TCACCTATCTAAACCTCAAACCA | |
| 102 | GGGGTTATTTGTTTAAGTTTTAAGTTAGT | ADHFE1 gene primers |
| 103 | AAACAATTACCTTCTACRACAATTTCA | |
| 104 | AAGTAAGGAGATTTAAGGTAGAATT | ADHFE1 gene primers |
| 105 | TTAAAATCCTCTTCCCTCCTC | |
| 106 | ATATGTGAAAAATAGAGTTGATAAGTAAGG | ADHFE1 gene primers |
| 107 | CTTAAAATCCTCTTCCCTCCTC | |
| 108 | GATGGTGCGAGCGTC | ADHFE1 gene primers |
| 109 | CTATCTAAACCTCAAACCAATCG | |
| 108 | GATGGTGCGAGCGTC | ADHFE1 gene primers |
| 110 | TACACCGCCCCCTCC | |
| 108 | GATGGTGCGAGCGTC | ADHFE1 gene primers |
| 111 | TCCGCCGACCAATCAC | |
| 108 | GATGGTGCGAGCGTC | ADHFE1 gene primers |
| 112 | CCGAACTCGAACGACAAC | |
| 113 | AYGGTAATTTTAAGGGTGGATGGTGYGAGY GT | ADHFE1 gene primers |
| 114 | CRCCCCCTCCRCCRACCAATCACRAAAACT | |
| 115 | AAGTAAGGAGATTTAAGGTAGAATTYGAGG TTTAYGGAGTAGT | ADHFE1 gene primers |
| 116 | ACCATAACRCTTAAAATCCTCTTCCCTCCTCR AATCRCT | |
| 117 | GGAGATTTAAGGTAGAATTCGAGGTTTAC | ADHFE1 gene primers |
| 118 | CTCGAACGACAACGACCATAAC | |
| 119 | GAATTCGAGGTTTACGGAGTAGT | ADHFE1 gene primers |
| 120 | CTCCTCGAATCGCTACACC | |
| 121 | TTTAAGGGTGGATGGTGCGAGC | ADHFE1 gene primers |
| 122 | CTCAACAAATACGCGACCC | |
| 123 | CGGTTAATTACGGAGGTTGTTC | ADHFE1 gene primers |
| 124 | CCTACGAAACAATTACCTTCTACGA | |
| 123 | CGGTTAATTACGGAGGTTGTTC | ADHFE1 gene primers |
| 125 | AAAACAAAACCCGAAACCTAC | |
| 126 | TAGTAAGTACGCGATTCGGGTTC | ADHFE1 gene primers |
| 127 | GAAACCTACGAAACAATTACCTTCTAC | |
| 128 | CGGAGTAGTTATTTTTTACGG | ADHFE1 gene primers |
| 129 | AACGACAACGACCATAAC | |
| 128 | CGGAGTAGTTATTTTTTACGG | ADHFE1 gene primers |
| 130 | GTTACAATTACCTCAACAAATACG | |
| 131 | GGTAGAATTCGAGGTTTACG | ADHFE1 gene primers |
| 132 | TCTTCCCTCCTCGAATCG | |
| 131 | GGTAGAATTCGAGGTTTACG | ADHFE1 gene primers |
| 133 | CAATCACGAAAACTACCCG | |
| 108 | GATGGTGCGAGCGTC | ADHFE1 gene primers |
| 134 | GAACTCGAACGACAACG | |
| 108 | GATGGTGCGAGCGTC | ADHFE1 gene primers |
| 135 | AACTCGAACGACAACGAC | |
| 108 | GATGGTGCGAGCGTC | ADHFE1 gene primers |
| 136 | CGTTACAATTACCTCAACAAATACG | |
| 108 | GATGGTGCGAGCGTC | ADHFE1 gene primers |
| 137 | TTAAAATCCTCTTCCCTCCTCG | |
| 108 | GATGGTGCGAGCGTC | ADHFE1 gene primers |
| 138 | ATCCTCTTCCCTCCTCG | |
| 139 | AGGTAGAATTCGAGGTTTACG | ADHFE1 gene primers |
| 140 | TACCCACCCGCTTCG | |
| 141 | TGGGAAAATGGTTTTGAGTTCG | ADHFE1 gene primers |
| 137 | TTAAAATCCTCTTCCCTCCTCG | |
| 142 | GAGGGAAGAGGATTTTAAGCG | ADHFE1 gene primers |
| 143 | CCAAAACGATATCCAAATTCTCCG | |
| 144 | GGATCGTAGGGTGTTTACG | ADHFE1 gene primers |
| 145 | CTACGCCCTAAAAAACTACATCG | |
| 146 | GAATTCGGAGAATTTGGATATCG | ADHFE1 gene primers |
| 147 | AACTCTCCACTACCTACTACG | |
| 148 | GCGTATTTGTTGAGGTAATTGTAACG | ADHFE1 gene primers |
| 149 | ACTCTATAACCTCGCTATTCCG | |
| 150 | AGGAGGGAAGAGGATTTTAAGCG | ADHFE1 gene primers |
| 151 | CCCAAAACGATATCCAAATTCTCCG | |
| 152 | GGGGATCGTAGGGTGTTTACG | ADHFE1 gene primers |
| 153 | CTCACTACGCCCTAAAAAACTACATCG | |
| 154 | TCGGTTAATTACGGAGGTTGTTCG | ADHFE1 gene primers |
| 155 | AACCTACGAAACAATTACCTTCTACG | |
| 156 | GAGTTTAGGGCGGTATTTAAATTTTTCG | ADHFE1 gene primers |
| 157 | AAACTCCAAACGCCATAACCG | |
| 158 | TTAAGCGTTATGGTCGTTGTCG | ADHFE1 gene primers |
| 159 | GCGCGATAAAAACCCAAAACG | |
| 160 | ACACGACAACGTCTATAAATATCGCCGAACC | OPLAH gene probe |
| 161 | CCCGACGATACTCATAAACGCTACCGTA | OPLAH gene probe |
| 162 | CCGTAACTACGTCCCCAAAACCGAAA | OPLAH gene probe |
| 163 | TCGGGTTTAGGAGGTCGTGTGAGG | OPLAH gene probe |
| 164 | AACCGTATAAAAATCCCGCAATAA | OPLAH gene probe |
| 165 | AAACGCAACTACGACGCCTAATTCGTAC | OPLAH gene probe |
| 166 | TTGGCGCGTATAGATACGATTCGGAGT | OPLAH gene probe |
| 167 | TAGTGCGTTTGTAGTTTCGAGT | OPLAH gene probe |
| 168 | CTCCCGATTCTAAAAACGCAA | OPLAH gene probe |
| 169 | ACGTAGTTACGGCGTTT | OPLAH gene probe |
| 170 | CAACGACCAAATACCCTCA | OPLAH gene probe |
| 171 | TTCGCGTCGTTCGTTATCGTTT | OPLAH gene probe |
| 172 | AGCGTGGTTCGAGTTTCGGTTT | OPLAH gene probe |
| 173 | ATTTGGTCGTTGCGTTGGGTTGG | OPLAH gene probe |
| 174 | CCCGCCACCGTCTCGTAAT | OPLAH gene probe |
| 175 | TTGCGAGGGCGAGGACGAGG | OPLAH gene probe |
| 176 | CGAGGACGAGGGTAAAGATTTAGCGT | OPLAH gene probe |
| 177 | AACGCAACGATATACACAACCACATAACCA AC | OPLAH gene probe |
| 178 | TTCGAGTTTCGGTTTTTATTCGGTCGTGT | OPLAH gene probe |
| 179 | TTCGGTTTTTATTCGGTCGTG | OPLAH gene probe |
| 180 | CACCGACCCTAAAATCCTAAAAAACCGAT | OPLAH gene probe |
| 181 | ACCGATAAACGACGTACGCGAAAACT | OPLAH gene probe |
| 182 | CTACGAAACGATAACGAACGACGCGAA | OPLAH gene probe |
| 183 | ACGAGGGTAAAGATTTAGCGT | OPLAH gene probe |
| 184 | CACACCGCTACGCCCGTACCAA | OPLAH gene probe |
| 185 | TGAGCGGCGTGCGCGGAG | OPLAH gene probe |
| 186 | AGTTTAGAGAGATTAGGGGCG | OPLAH gene probe |
| 187 | TACGCGATTCCCCAACCCTCGCGAC | OPLAH gene probe |
| 188 | AAGGCGCGGCGTTCGGTTAG | OPLAH gene probe |
| 189 | TCAAAACCATTTTCCCACGAAATAAA | ADHFE1 gene probe |
| 190 | CTCTATAACCTCGCTATTCCGCCT | ADHFE1 gene probe |
| 191 | CCCACGAAATAAAAACCAAAACGCCAAC | ADHFE1 gene probe |
| 192 | ACTCAAAACCATTTTCCCACGAA | ADHFE1 gene probe |
| 193 | ACTCAAAACCATTTTCCCACGAAAT | ADHFE1 gene probe |
| 194 | AACGAAAAACGCCACTACCCGATACGA | ADHFE1 gene probe |
| 195 | AACGCCACTACCCGATACGAACTACACC | ADHFE1 gene probe |
| 196 | AAAAACCAAAACGCCAACTACCCCA | ADHFE1 gene probe |
| 197 | CAAAACGCCAACTACCCCAACGAC | ADHFE1 gene probe |
| 198 | TGCGAGCGTCGTTGGGGTA | ADHFE1 gene probe |
| 199 | TGGTCGTTGTCGTTCGAGTTCGG | ADHFE1 gene probe |
| 200 | CCTCGCTATTCCGCCTAACGCG | ADHFE1 gene probe |
| 201 | ACTACGCAAAATCTAAACGAATCA | ADHFE1 gene probe |
| 202 | TATGGTCGTTGTCGTTCGAGTT | ADHFE1 gene probe |
| 203 | TACGTAAACACCCTACGATCCC | ADHFE1 gene probe |
| 204 | TTCGTTTAGATTTTGCGTAG | ADHFE1 gene probe |
| 205 | AACGATATCCAAATTCTCCGAAT | ADHFE1 gene probe |
| 206 | TTCGTTTTTCGGTCGCGTTTGTT | ADHFE1 gene probe |
| 207 | ATACGAACGCCGCTAAAACAACTAACGTTCT | ADHFE1 gene probe |
| 208 | TACCGCCCGAACCCGAATCGCGTA | ADHFE1 gene probe |
| 209 | AAGCGGAGAAGATTGCGTA | ADHFE1 gene probe |
| 3 | GAATTAGGCGTCGTAGTTGC | OPLAH gene primers |
| 4 | CGCAAACACTAACCTTTCCC | |
| 5 | GGGTTCGGCGATATTTATAGAC | OPLAH gene primers |

As used herein, the inventors use a sampling device to collect stool samples, and the samples undergo nucleic acid extraction and purification steps to obtain nucleic acid samples containing human DNA. Further, the inventors use bisulfite to treat the nucleic acid sample to convert unmethylated cytosine into uracil, so that the methylation site can be detected. For the gene, the inventors designed several primers and probes as described in Table 1, which can specifically amplify and identify relevant sites. The primer and probe sequences are continuous fragments of at least 15 bases in length, and the nucleic acid sequences of the primers and probes are equivalent, complementary or hybridized to the methylated region after methylation modification. By using the fluorescent quantitative PCR method, the primers and probes provided herein can effectively reflect the methylation status of the relevant sites through Ct values and/or △Ct values (the difference between the Ct value of the target gene and the Ct value of the internal reference gene) and/or curve conditions, and then the methylation status of the relevant genes of the subject is judged. Furthermore, by detecting samples of several cancer patients and non-cancer patients, a cutoff value for determining the risk of cancer can be obtained. By comparing the detection results with the cutoff value, the risk of cancer in the subject can be evaluated.

The optimized primers, probes and PCR reaction system can form a methylation detection kit for OPLAH gene and ADHFE1 gene, and the kit can further be used for cancer risk assessment.

About fecal occult blood detection:
The hemoglobin in the fecal sample is detected, and the detection method may include fecal immunochemical test (FIT), guaiac test (gFOBT), tetramethylbenzidine method and ELISA double antibody sandwich method.

In the fecal occult blood detection method, FIT uses a monoclonal or polyclonal antibody to directly detect hemoglobin in human feces, and is not affected by food intake. In qualitative FIT, a visible color change appears when fecal hemoglobin content exceeds a certain threshold; while in quantitative FIT, a value can be measured, and when it exceeds a certain normal value range, positive is defined. Qualitative FIT can be performed by using the immunocolloidal gold method, which has the advantages of convenient and fast testing; while quantitative FIT can be performed by using the latex immunoturbidimetry method, which has high sensitivity and specificity.

About the method for combination of gene detection and fecal occult blood detection:
The method for result judgement of the combined detection of gene and fecal occult blood may comprise that the judgement thresholds can be customized for the gene detection results and the fecal occult blood detection, respectively, and the subject is determined to be positive or a positive candidate based on the above two parts of the detection results.

The judgement method can also be a logistic regression method, in which different weights are assigned to the detection results of OPLAH gene, ADHFE1 gene and fecal occult blood, and calculation is carried out through a logistic function. The logistic function is g(y)=1/(1+e^{-y}), wherein g(y) is the risk index, and when the risk index is higher than a preset value, the subject is judged to be positive or a positive candidate; e is the natural constant; y=(θ0+θ1*X1+θ2*X2+...+θn*Xn), wherein θ0 is a constant term, θi (i=1, 2, ..., n) is a coefficient to be determined, and Xi (i=1, 2, ..., n) is the result of gene and fecal occult blood detection. Different weights, i.e., θi values, reflect different contributions of independent variables to dependent variables. Different detection results, i.e., Xi values, may include but are not limited to one or any combination of more of the Ct value, △Ct value, methylation rate of gene detection, the fecal occult blood qualitative detection result and the fecal occult blood quantitative detection result.

By optimizing the parameters in the judgement method, higher detection sensitivity can be obtained.

The kit comprises a combined detection reagent for methylation of OPLAH and ADHFE1 genes based on fluorescent quantitative PCR, which can provide methylation detection results of OPLAH and ADHFE1 genes. Furthermore, the kit may also comprise a fecal occult blood detection reagent, which can provide fecal occult blood detection results. By calculating the △Ct value or risk index and comparing it with a preset positive judgment value of the kit, it can be used to judge the risk of colorectal cancer.

The combined detection reagent for methylation of OPLAH and ADHFE1 gene based on fluorescent quantitative PCR method comprises primers and probes that can specifically amplify OPLAH and ADHFE1 genes. The reagent can detect the methylation status of OPLAH and ADHFE1 genes in the nucleic acid after methylation modification.

The combined detection reagent for gene methylation can be used with different stool sample sampling devices, nucleic acid extraction reagents and methylation modification pretreatment reagents. The stool sampling device may comprise a sampler and a sample storage device for collecting and storing sample. The nucleic acid extraction reagent can be a kit that can extract a nucleic acid from a human biological sample, or a kit containing magnetic beads for capturing a nucleic acid. The methylation modification pretreatment reagent modifies the nucleic acid sequence so that a difference is generated between the methylated cytosine and the unmethylated cytosine, which is easy to detect. Generally, the methylation modification pretreatment reagent can convert cytosine that has not undergone methylation modification into uracil, while cytosine that has undergone methylation modification remains unchanged, and the sequence change can be used for subsequent detection.

The fecal occult blood detection reagent can be preferably used for the immunocolloidal gold method to obtain fecal occult blood qualitative detection results simply and quickly. The immunocolloidal gold method fecal occult blood detection reagent may comprises a sampler, a sample storage device and a test strip. The test strip is coated with antibody and colloidal gold. When the target protein labeled with colloidal gold binds to the pre-coated antibody on the test strip, it will be retained on the test strip to form a visual change, thereby judging whether the sample contains the target protein.

The positive judgment value is delineated using samples with known clinical information. By detecting a number of positive and negative samples, a classification model is constructed to determine the conditions for positive judgment.

Another aspect of the present invention relates to a diagnosis or evaluation model for colorectal cancer, in which calculation is carried out using a logical function g(y)=1/(1+e^{-y}), wherein:
g(y) is a risk index, and when the risk index is higher than a preset value, it is judged to be positive or a positive candidate;
e is the natural constant;
y=(θ0+θ1*X1+θ2*X2+...+θn*Xn), θ0 is a constant term, θi (i=1, 2, ..., n) is a coefficient to be determined, and Xi (i=1, 2, ..., n) is the detection result of the differentially methylated region and the detection result of fecal occult blood in any one of items of the present invention.

Another aspect of the present invention relates to a system for diagnosis or evaluation of colorectal cancer, comprising:
1) a DMR detection unit: which is configured to obtain content data of the differentially methylated region as described in any one of items of the present invention or a fragment thereof; wherein the fragment comprises one or more methylation sites in the differentially methylated region;
2) a fecal occult blood detection unit: which is configured to obtain fecal occult blood detection result data;
3) a data analysis unit: which is configured to input the data obtained by the DMR detection unit and the fecal occult blood detection unit into the colorectal cancer diagnosis or evaluation model of the present invention for analysis;
4) a risk assessment unit: which is configured to output a risk index or judgment result of an individual sample.

Another aspect of the present invention relates to the use of the differentially methylated region (1) and the differentially methylated region (2) of the present invention in the manufacture of a product for detecting a colorectal cancer or a colorectal cancer precancerous lesion.

In the present invention, if not otherwise specified, the term "colorectal cancer" refers to colon cancer and/or rectal cancer, also briefly referred to as "intestinal cancer".

In the present invention, if not otherwise specified, the term "early colorectal cancer" refers to stage I or stage II colorectal cancer.

In the present invention, if not otherwise specified, detecting a differentially methylated region or detecting a fragment thereof refers to detecting the content of a differentially methylated region or detecting the content of a fragment thereof; wherein the fragment comprises one or more methylation sites within the differentially methylated region.

In the present invention, if not otherwise specified, detecting a methylation site within a differentially methylated region refers to detecting the content of a methylation site within a differentially methylated region or detecting the methylation rate of a methylation site within a differentially methylated region.

### Beneficial effects of the present invention

The present invention achieves one or more of the following technical effects:
(1) The present invention is capable of realizing rapid detection of the methylation status of OPLAH gene and ADHFE1 gene;
(2) The present invention is capable of detecting, diagnosing or screening colorectal cancer, or being used for assessing the prognostic risk of colorectal cancer, with high sensitivity and/or specificity; preferably, with both high sensitivity and specificity;
(3) The present invention is capable of being used for the detection, diagnosis or screening of early colorectal cancer or colorectal precancerous lesions, with high sensitivity and/or specificity; preferably, with both high sensitivity and specificity;
(4) The present invention is capable of performing combined detection of hemoglobin in feces to further improve the detection sensitivity of colorectal cancer;
(5) Compared with traditional detection methods, the present invention has the advantages of higher sensitivity, completely non-invasive, non-invasive detection process, no need for intestinal preparation, and high acceptance of detection by asymptomatic people.

### Brief Description of the Drawings

Figure 1 shows the differential methylation regions of OPLAH and ADHFE1 genes in colorectal cancer and para-carcinoma tissue samples.
Figure 2 shows the reference results of OPLAH and ADHFE1 gene detection.
Figure 3 shows the ROC curves of clinical samples in OPLAH and ADHFE1 gene detection.
Figure 4 shows the comparison of ROC curves of gene detection and gene + FIT joint detection.
Figure 5 shows the comparison of detection sensitivity.

### Specific Models for Carrying Out the present invention

The technical solution of the present invention will be described in detail in conjunction with the examples, but those skilled in the art will understand that the following examples are only used to illustrate the present invention and should not be regarded as limiting the scope of the present invention. If the specific conditions were not specified in the examples, they were carried out according to conventional conditions or conditions recommended by the manufacturer. The reagents or instruments used without indicating the manufacturer were all conventional products that could be purchased commercially.

Some relevant definitions refer to the Colorectal Cancer Diagnosis and Treatment Guidelines 2021, the Chinese Society of Clinical Oncology (CSCO), and some relevant contents are quoted as follows in Table 2:

**Table 2**

| | |
|---|---|
| Colorectal cancer | Colon cancer or rectal cancer confirmed by biopsy pathological diagnosis. |
| Advanced adenoma | Carcinoma in situ and intramucosal carcinoma, with diameter >= 1 cm, or with villous structure, or with high-grade neoplasia, with severe dysplasia. |
| Adenoma patients | Patients with adenoma confirmed by pathological diagnosis. |
| Polyp patients | Patients with inflammatory polyps or hyperplastic polyps confirmed or unconfirmed by pathological diagnosis. |
| Other intestinal disease patients | Including patients with non-polyp lesions such as enteritis, diverticulum, and colon melanosis. |

The standard for staging colorectal cancer adopts AJCC.

### Example 1: Combined detection of methylation levels of OPLAH and ADHFE1 genes by real-time fluorescence quantitative PCR

The inventors discovered differentially methylated regions of OPLAH and ADHFE1 genes through targeted methylation capture sequencing. The differentially methylated regions had high methylation levels in cancer tissues, while low methylation levels in para-cancerous tissues. In this example, by taking fluorescence quantitative PCR method as an example, for the regions, the inventors designed several methylation-specific primers and probes that could specifically amplify methylated nucleic acid sequences. After system primer screening and system optimization, a combined detection method for OPLAH and ADHFE1 genes based on fluorescence quantitative PCR was developed.

The specific steps were as follows:
1. Discovery of differentially methylated regions by targeted methylation capture sequencing:
   For 38 pairs of colorectal cancer sample and para-cancerous tissue sample from 38 people (wherein, each pair of colorectal cancer sample and para-cancerous tissue sample came from the same person), nucleic acids were extracted using DNeasy Blood & Tissue Kit (Qiagen, #69506) and quantified using Qubit 3.0 system (Invitrogen, USA).

200ng of extracted DNA was taken, added at the same time with 1ng of Unmethylated lambda DNA (PROMEGA, #D1521) for subsequent C-U conversion quality control; an ultrasonic shearing instrument was used to break the DNA, and then AMPure XP (AGENCOURT, #A63882) was used to select DNA fragments so that the DNA fragment size was concentrated around 160bp.

KAPA HyperPlus Library Preparation Kit (KAPA, #KK8510) was used to construct a library, and EZ DNA Metlylation-Gold Kit (Zymo Research, #D5006) was used to perform the bisulfite conversion of adapter ligation product. The specific operations were referred to the kit instructions, in which the adapters used in the adapter ligation step and the PCR primers used in the PCR step were replaced with adapters and primers suitable for the MGISEQ platform, and the adapters needed to be methylated (for example, methylated dCTP was used to synthesize the adapters) to avoid sequence changes caused by bisulfite treatment.

Seq Cap EZ Hybridization and Wash Kit (ROCHE, 5634253001) and SeqCap Epi CpGiant Enrichment Kit (ROCHE, 7138911001) were used for hybridization, capture, and elution. Since the sequencing instrument of the MGI platform was used, the blocking of the adapter sequences during the hybridization process must use the blocking sequences corresponding to the MGI sequencing platform.

PE100 sequencing was performed using MGISEQ-2000 (MGI).

After quality control of sequencing data, filtering of unqualified sequencing sequences, and alignment of sequenced sequences with the computer-simulated bisulfite conversion form of human genome sequences, all sequences were counted and the methylation rate of each CpG site was calculated (Non-invasive lung cancer diagnosis and prognosis based on multi-analyte liquid biopsy, Chen Kezhong et al., 2020).

Specific site methylation rate = methylation reads sequencing depth / total sequencing depth.

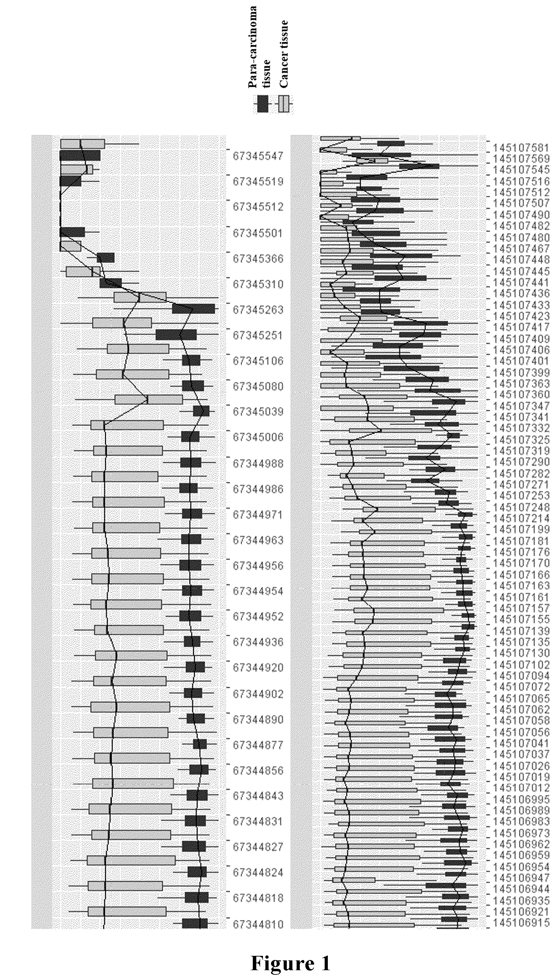

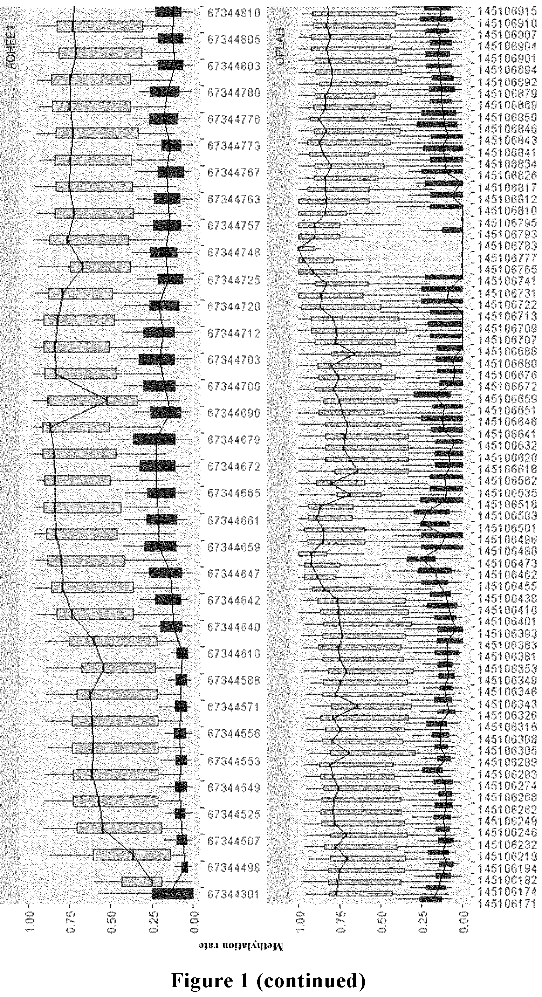

The inventors found that there were significant differentially methylated regions in the OPLAH gene and the ADHFE1 gene, which had the potential to distinguish cancer from non-cancer. Figure 1 showed the methylation levels of each CpG site in the OPLAH and ADHFE1 genes in cancer tissues and para-cancerous tissues.

### 2. Design of methylation primers and probes

Based on the differentially methylated regions found, the inventors designed primers and probes that could specifically amplify nucleic acid sequences pretreated with methylation modification for CpG sites with high methylation rates in cancer tissues and low methylation rates in para-cancerous tissues, wherein at least one of the primers in a pair of primers had at least one CpG site at its 3' end. The designed primer and probe sequences were shown in Table 1.

### 3. Fluorescence quantitative PCR capable of performing combined detection of DMR methylation levels of OPLAH and ADHFE1 genes

The inventors selected the optimized primer sets and probes for methylation detection of OPLAH and ADHFE1 genes. The primer sets described in this example included: OPLAH gene primer set (SEQ ID NO: 30 and SEQ ID NO: 32), OPLAH gene probe (SEQ ID NO: 170), ADHFE1 gene primer set (SEQ ID NO: 108 and SEQ ID NO: 109) and ADHFE1 gene probe (SEQ ID NO: 197). The fluorescence quantitative PCR reaction system comprised 1× PCR Buffer, 2 to 5 mM magnesium ions, 0.2 to 0.8 mM dNTP, 0.2 µM primer for each gene, 50 nM probe for each gene, and 1 unit of Taq polymerase. The reaction comprised pre-denaturation at 95°C for 10min, and then 40 cycles of denaturation at 95°C for 30 seconds, annealing at 55°C for 30 seconds and extension at 72°C for 30 seconds. The detection was performed using Hongshi 96S qPCR instrument, with the baseline and threshold set as default, and the fluorescence signal was collected at the end of each cycle.

The inventors used a fully methylated standard and a fully unmethylated standard (Merck, #S8001) to verify the ability of the method to detect the methylation levels of OPLAH and ADHFE1 genes. The fully methylated standard was a cell line DNA treated with M. SssI methyltransferase, and all CpG sites were methylated; the fully unmethylated standard was a methyltransferase gene-knocked cell line DNA, and the methylation level of its CpG sites was less than 5%. The inventors used these two standards and mixed them to form references with different methylation levels, including 100%, 50%, 25%, 12%, 6%, 3%, 1% and 0%.

20ng of each reference was taken to perform detection 3 times. First, the DNA was subjected to the pretreatment of methylation modification using the methylation detection sample pretreatment kit (BGI) to convert the unmethylated cytosine into uracil. Then, the reference was detected using the above-mentioned fluorescent quantitative PCR reaction system.

The detection results were shown in Table 3.

**Table 3: Data of references for detection of OPLAH and ADHFE1 genes**

| Methylation rate | DMR of OPLAH | | | | DMR of ADHFE1 | | | |
|---|---|---|---|---|---|---|---|---|
| | Ct | | | Average Ct | Ct | | | Average Ct |
| 100% | 28.43 | 28.31 | 28.3 | 28.35 | 28.44 | 28.47 | 28.61 | 28.51 |
| 50% | 29.12 | 29.14 | 29.3 | 29.19 | 29.81 | 29.58 | 29.77 | 29.72 |
| 25% | 30.56 | 30.4 | 30.55 | 30.50 | 30.83 | 30.9 | 30.91 | 30.88 |
| 13% | 31.3 | 31.33 | 31.2 | 31.28 | 32.6 | 32.67 | 32.55 | 32.61 |
| 6% | 32.42 | 32.35 | 32.57 | 32.45 | 33.05 | 33.21 | 34.34 | 33.53 |
| 3% | 33.17 | 32.8 | 33.45 | 33.14 | 36.12 | 35.62 | 34.91 | 35.55 |
| 1% | 34.1 | 34.3 | 34.5 | 34.30 | 35.87 | 35.94 | 36 | 35.94 |
| 0% | NoCt | NoCt | NoCt | / | NoCt | NoCt | NoCt | / |

The results showed that all references mixed with methylated templates were detected, with a detection limit as low as 1%. However, no amplification occurred in the references without methylated templates.

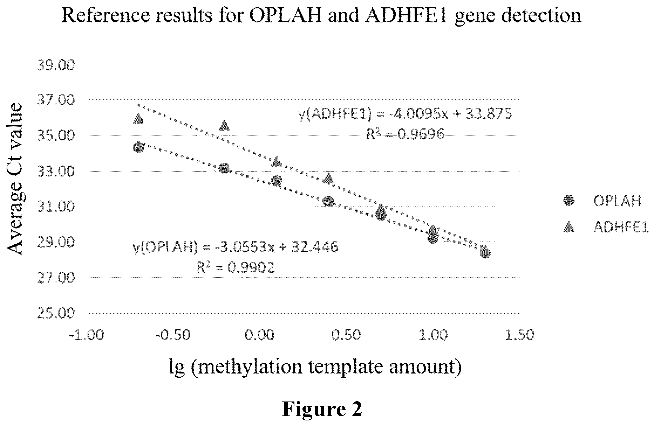

Based on the amounts of methylated templates as loaded and the average Ct values, a standard curve was drawn. As shown in Figure 2, the R² of the standard curve was good. This showed that the method of the present invention could effectively detect the methylation signals of OPLAH and ADHFE1 genes in the samples, and there was no non-specific amplification.

### Example 2: Combined detection kit of methylation of OPLAH and ADHFE1 genes for colorectal cancer detection

In this example, the fluorescent quantitative PCR method was taken as an example to disclose a kit for colorectal cancer detection by combined detection of methylation levels of OPLAH and ADHFE1 genes.

This kit comprised reagents such as buffer, dNTP and enzyme required for PCR amplification, and also comprised primer sets and probes of OPLAH and ADHFE1 genes. The primer sets described in this example included: OPLAH gene primer set (SEQ ID NO: 30 and SEQ ID NO: 32), OPLAH gene probe (SEQ ID NO: 170), ADHFE1 gene primer set (SEQ ID NO: 108 and SEQ ID NO: 109) and ADHFE1 gene probe (SEQ ID NO: 197). In order to monitor whether the sample contained sufficient human DNA, primers and probes for detecting internal reference gene were also added to the kit. The reference gene was generally a housekeeping gene, and its corresponding primer and probe detection results were only related to the total amount of human DNA, and were not affected by the methylation level. In this example, GAPDH gene was selected as the reference gene, and its primer and probe sequences were shown in Table 4. The PCR reaction comprised pre-denaturation at 95°C for 10 minutes, and then 40 cycles of denaturation at 95°C for 30 seconds, annealing at 55°C for 30 seconds and extension at 72°C for 30 seconds. The detection was performed using Hongshi 96S qPCR instrument,with the baseline and threshold set at default, and the fluorescence signal was collected at the end of each cycle.

**Table 4: Sequence table of internal reference gene primers and probes**

| SEQ ID NO: | Sequence (5'-3') | Description |
|---|---|---|
| 210 | TTTAGGAGTGAGTGGAAGATAGA | Internal reference gene primers |
| 211 | AAACCACACCATCCTAATTACCT | |
| 212 | CCCAAAACACATTTCTTCCATTC | Internal reference gene probe |

A total of 604 stool samples were included in the present invention to evaluate the performance of the kit, and the information of clinical samples was shown in Table 5.

**Table 5: Sample information table**

| Sample type | Colorectal cancer patients | Healthy people | Polyp patients | Adenoma patients | Patients with other intestinal diseases | Advanced adenoma patients | Total |
|---|---|---|---|---|---|---|---|
| Number of samples | 284 | 146 | 49 | 55 | 13 | 57 | 604 |
| Median age (years) | 59 (32-84) | 63 (29-78) | 64 (37-74) | 65 (30-76) | 56 (49-86) | 63 (38-75) | 62 (29-86) |

| Gender (n) | | | | | | | |
|---|---|---|---|---|---|---|---|
| Male | 170 | 61 | 29 | 29 | 10 | 25 | 324 |
| Female | 114 | 85 | 20 | 26 | 3 | 32 | 280 |

| Colorectal cancer stage | | | | | | | |
|---|---|---|---|---|---|---|---|
| Stage I | 44 (15.49%) | | | | | | |
| Stage II | 82 (28.87%) | | | | | | |
| Stage III | 78 (27.46%) | | | | | | |
| Stage IV | 22 (7.75%) | | | | | | |
| Unknown | 58 (20.42%) | | | | | | |

Sample detection: First, stool samples were collected from the subjects using disposable stool sample samplers (BGI). Then, stool sample DNA was extracted using a stool sample nucleic acid extraction kit (BGI). Next, the DNA was pre-treated with a methylation detection sample pretreatment kit (BGI) to obtain a converted DNA. Finally, the kit of the present invention was used to detect the converted DNA to obtain the Ct values of the internal reference, OPLAH and ADHFE1 genes of each sample.

When analyzing the results, the threshold of the internal reference gene was 37. When the Ct value of the sample internal reference gene was greater than 37, it was considered that the human DNA content in the sample was insufficient and the detection was judged to have failed. When the Ct value of the internal reference gene was less than or equal to 37, the sample was judged to have passed quality inspection. For samples that passed the quality inspection, when the Ct value of the target gene was greater than 38 or no Ct value was detected, the gene detection was judged to have negative result. When the Ct value of the target gene was less than or equal to 38, the △Ct value of the target gene (the difference between the Ct value of the target gene and the Ct value of the reference gene) was further calculated.

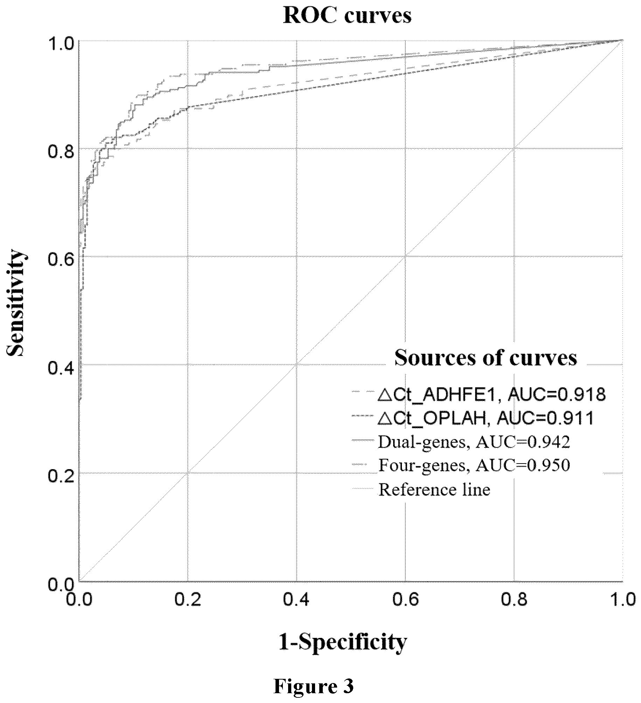

The positive cutoff value is determined by SPSS analysis. First, the inventors used SPSS software to draw the ROC curve for a single gene. As shown in Figure 3, the AUC values of OPLAH and ADHFE1 genes were 0.911 and 0.918, respectively. By integrating the detection results of the two genes for analysis, a higher AUC of 0.942 was obtained. Furthermore, the inventors used the same method to detect the PPP2R5C and SDC2 genes of the above samples, and obtained the Ct values and △Ct values of the PPP2R5C and SDC2 genes. The PPP2R5C and SDC2 genes also had significant methylation differences between cancer tissues and non-cancerous tissues. Further, the inventors used the SPSS software to analyze the detection effect of the four-gene combination of OPLAH, ADHFE1, PPP2R5C and SDC2. The detection results were shown in Figure 3. It could be seen that the detection effect of the four-gene combination detection was similar to that of the OPLAH and ADHFE1 double gene detection, and the AUC was 0.950. Since the double gene detection could achieve the effect of the four-gene combination detection, considering the cost and detection stability, the method of using the double gene detection was determined. It was finally determined that when the △Ct value of the DMR of OPLAH gene was less than or equal to 7, or the △Ct value of the DMR of ADHFE1 gene was less than or equal to 12, the clinical sample detection performance was the best. The specific performance was shown in Table 6. The sensitivity of the kit of the present invention reached 86.62%, the overall specificity (specificity for total of healthy samples and polyp samples) reached 92.78%, and the sensitivity for advanced adenoma reached 52.63%. The kit of the present invention also had a high sensitivity for early-stage colorectal cancer. As shown in Table 7, the sensitivity for stage I and stage II colorectal cancer reached 88.64% and 89.02%, respectively.

**Table 6: Detection performance in clinical samples**

| Sample type | Number of samples with positive detection results | Number of samples with negative detection result | Total number of samples | Sensitivity | Specificity |
|---|---|---|---|---|---|
| Patients with colorectal cancer | 246 | 38 | 284 | 86.62% | |
| Healthy people | 11 | 135 | 146 | / | 92.47% |
| Patients with polyps | 1 | 48 | 49 | / | 97.96% |
| Patients with adenoma | 5 | 50 | 55 | / | 90.91% |
| Patients with other intestinal diseases | 2 | 11 | 13 | / | 84.62% |
| Patients with advanced adenoma | 30 | 27 | 57 | 52.63% | / |
| Total | 295 | 309 | 604 | 86.09% (total coincidence rate) | |

wherein, total coincidence rate = (number of samples with correct gold standard negative detection result + number of samples with correct gold standard positive detection result) / total number of samples. The gold standard referred to colonoscopy.

**Table 7: Detection sensitivity for colorectal cancer samples at different stages**

| Sample stage | Number of samples with positive detection results | Total number of samples | Sensitivity |
|---|---|---|---|
| Stage I | 39 | 44 | 88.64% |
| Stage II | 73 | 82 | 89.02% |
| Stage III | 71 | 78 | 91.03% |
| Stage IV | 18 | 22 | 81.82% |
| Unknown | 45 | 58 | 77.59% |
| Total | 246 | 284 | 86.62% |

According to the above method, the inventors also used some other primer and probe combinations in Table 1 to perform detection on the above samples, and all of them brought good detection results. The specific results were shown in Table 8 below.

**Table 8**

| Primer sequence No. | Probe sequence No. | Sensitivity for colorectal cancer | Sensitivity for advanced adenoma | Specificity for total of healthy samples and polyp samples |
|---|---|---|---|---|
| 25, 26, 144 and 145 | 164, 204 | 83.45% | 50.88% | 88.21% |
| 26, 27, 154 and 155 | 168, 206 | 85.21% | 38.60% | 89.73% |
| 33, 34, 156 and 157 | 171, 209 | 71.13% | 36.84% | 91.63% |
| 56, 57, 158 and 159 | 184, 203 | 88.03% | 56.14% | 80.99% |
| 61, 62, 108 and 109 | 183, 191 | 84.51% | 43.86% | 88.97% |
| 33, 34, 108 and 109 | 171, 191 | 79.23% | 42.11% | 90.11% |
| 25, 26, 158 and 159 | 164, 203 | 75.35% | 36.84% | 90.49% |

### Example 3: Kit for colorectal cancer detection by combined detection of OPLAH and ADHFE1 gene methylation and fecal occult blood

The present invention disclosed a kit for colorectal cancer detection by combined detection of OPLAH and ADHFE1 gene methylation levels and fecal occult blood. The kit comprised a module for detection of OPLAH and ADHFE1 gene methylation levels and a module for detection of fecal occult blood.

### 1. Module for detection of OPLAH and ADHFE1 gene methylation levels

The module for detection of OPLAH and ADHFE1 gene methylation levels was based on the fluorescent quantitative PCR method, and comprised reagents such as buffer, dNTP and enzymes required for PCR amplification, and also comprised primer sets and probes for OPLAH and ADHFE1 genes. The primer sets as described in this example could be selected from Table 1. The primer sets selected for this example included: OPLAH gene primer set (SEQ ID NO: 30 and SEQ ID NO: 32), OPLAH gene probe (SEQ ID NO: 170), ADHFE1 gene primer set (SEQ ID NO: 108 and SEQ ID NO: 109) and ADHFE1 gene probe (SEQ ID NO: 197). In order to monitor whether the sample contained enough human DNA, primers and probes for detecting internal reference gene were also added to this kit. The internal reference gene was generally a housekeeping gene, and the corresponding primer and probe detection results were only related to the total amount of human DNA, and were not affected by the methylation levels. In this example, the GAPDH gene was selected as the internal reference gene, and its primer and probe sequences were shown in Table 4. The PCR reaction comprised pre-denaturation at 95°C for 10 min, and then 40 cycles of denaturation at 95°C for 30 seconds, annealing at 55°C for 30 seconds and extension at 72°C for 30 seconds. The detection was performed using Hongshi 96S qPCR instrument with the baseline and threshold set as default, and the fluorescence signal was collected at the end of each cycle.

Sample detection: First, a disposable stool sample sampler (BGI) was used to collect a fecal sample of the subjects. Then, a fecal sample nucleic acid extraction kit (BGI) was used to extract a fecal sample DNA. Next, the DNA was pre-treated with methylation modification using a methylation detection sample pretreatment kit (BGI) to obtain a converted DNA. Finally, the converted DNA was detected using this kit to obtain the Ct values of the internal reference, OPLAH and ADHFE1 genes of each sample.

When analyzing the results, the threshold of the internal reference gene was 37. When the Ct value of the sample internal reference gene was greater than 37, it was considered that the human DNA content in the sample was insufficient and the detection was judged to have failed. When the Ct value of the internal reference gene was less than or equal to 37, the sample was judged to have passed quality inspection. For samples that had passed the quality inspection, when the Ct value of the target gene was greater than 38 or no Ct value was detected, the gene was judged to have negative detection result. When the Ct value of the target gene was less than or equal to 38, the △Ct value of the target gene (the difference between the Ct value of the target gene and the Ct value of the internal reference gene) was further calculated.

### 2. Module for detection of fecal occult blood

The module for detection of fecal occult blood comprised a test strip that could qualitatively detect the content of fecal hemoglobin, and its detection principle was the colloidal gold method. Specifically, a fecal sample was collected using a FIT sampler, mixed with a sample preservation solution, and about 100µl of the sample was taken on the test strip, and the detection result was read within a certain period of time.

When analyzing the results, a band should appear on the quality control line, otherwise the detection failed; if the quality control line was qualified, the test line was observed. If a band appeared on the test line, the fecal occult blood detection was positive, and if there was no band, it was negative. The result reading should be completed within the specified time, and should be invalid when the time exceeded the specified time.

### 3. Implementation and performance evaluation of kit for OPLAH and ADHFE1 gene methylation detection combined with fecal occult blood detection

In this example, 318 samples with fecal occult blood detection results from the samples described in Example 2 were used for analysis, and the clinical information of the samples was shown in Table 9.

**Table 9: Sample information table**

| Sample type | Colorectal cancer patients | Healthy people | Polyp patients | Adenoma patients | Patients with other intestinal diseases | Advanced adenoma patients | Total |
|---|---|---|---|---|---|---|---|
| Number of samples | 149 | 96 | 21 | 21 | 2 | 29 | 318 |
| Median age (years) | 59 (38-82) | 63 (29-78) | 64 (42-72) | 63 (30-74) | 55 (54-56) | 62 (40-75) | 61 (29-82) |

| Gender (n) | | | | | | | |
|---|---|---|---|---|---|---|---|
| Male | 84 | 41 | 9 | 15 | 1 | 14 | 164 |
| Female | 65 | 55 | 12 | 6 | 1 | 15 | 154 |

| Colorectal cancer stage | | | | | | | |
|---|---|---|---|---|---|---|---|
| Stage I | 19 (12.75%) | | | | | | |
| Stage II | 32 (21.48%) | | | | | | |
| Stage III | 45 (30.2%) | | | | | | |
| Stage IV | 13 (8.72%) | | | | | | |
| Unknown | 40 (26.85%) | | | | | | |

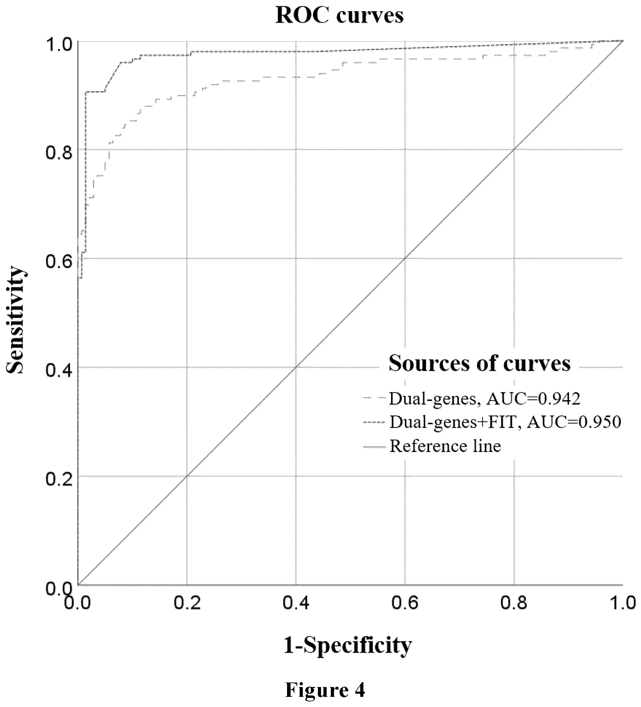

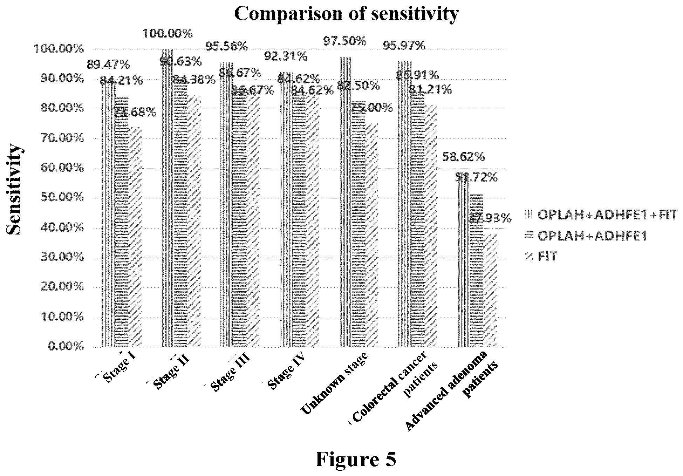

The positive cutoff value was determined by SPSS analysis. First, the inventors used SPSS software to draw the ROC curve for the gene detection results. As shown in Figure 4, the AUC value of the combined detection of OPLAH and ADHFE1 genes was 0.931. OPLAH and ADHFE1 genes had good detection performance in colorectal cancer detection. Furthermore, the inventors incorporated the results of fecal occult blood detection (FIT) into the logistic regression analysis, and calculated the detection risk index according to the formula: g(y)=1/(1+e^{-y}), wherein, g(y) was the risk index. When the risk index was higher than the preseted value, it was judged to be positive or a positive candidate; e was the natural constant; y=(θ0+θ1*X1+θ2*X2+...+θn*Xn), θ0 was a constant term, θi (i=1, 2, ..., n) was the coefficient to be determined, and Xi (i=1, 2, ..., n) was the result of gene and fecal occult blood detection. After calculating the detection risk index of each sample, the ROC curve was drawn to determine the optimal positive cutoff value. As shown in Figure 4, the AUC of the combined detection of gene and FIT reached 0.975, and the combined detection of gene and FIT could effectively improve the detection performance. As shown in Table 10, when the positive cutoff value was set to the detection risk index of 0.55, the best performance was achieved. At this time, the sensitivity and overall specificity were 95.97% and 91.43%, respectively. This kit had a good detection rate for advanced adenoma, and reached a sensitivity of 58.62%. This kit had a high sensitivity for early colorectal cancer. As shown in Table 11, the sensitivity for stage I and stage II colorectal cancer was 89.47% and 100%, respectively. As shown in Figure 5, compared with the dual gene detection or FIT detection, this kit had a higher detection sensitivity for both colorectal cancer and advanced adenoma.

**Table 10: Detection performance for clinical samples**

| Sample type | Number of samples with positive detection results | Number of samples with negative detection result | Total number of samples | Sensitivity | Specificity |
|---|---|---|---|---|---|
| Patients with colorectal cancer | 143 | 6 | 149 | 95.97% | / |
| Healthy people | 4 | 92 | 96 | / | 95.83% |
| Patients with polyps | 2 | 19 | 21 | / | 90.48% |
| Patients with adenoma | 5 | 16 | 21 | / | 76.19% |
| Patients with other intestinal diseases | 1 | 1 | 2 | / | 50.00% |
| Patients with advanced adenoma | 17 | 12 | 29 | 58.62% | / |
| Total | 172 | 146 | 318 | 90.57% (total coincidence rate) | |

wherein, total coincidence rate = (number of samples with correct gold standard negative detection result + number of samples with correct gold standard positive detection result) / total number of samples. The gold standard referred to colonoscopy.

**Table 11: Detection sensitivity for colorectal cancer samples at different stages**

| Sample stage | Number of samples with positive detection results | Total number of samples | Sensitivity |
|---|---|---|---|
| Stage I | 17 | 19 | 89.47% |
| Stage II | 32 | 32 | 100.00% |
| Stage III | 43 | 45 | 95.56% |
| Stage IV | 12 | 13 | 92.31% |
| Unknown | 39 | 40 | 97.50% |
| Total | 143 | 149 | 95.97% |

Although the specific embodiments of the present invention have been described in detail, those skilled in the art will understand that various modifications and substitutions can be made to those details based on all the teachings disclosed, and these changes are within the scope of protection of the present invention. The full scope of the present invention is given by the appended claims and any equivalents thereof.

## Claims

1. A differentially methylated region, comprising:
(1) chr8: 145106171-145107467, and
(2) chr8:67344198-67345563.

2. The differentially methylated region according to claim 1, wherein:
the differentially methylated region shown in item (1) has a base sequence as set forth in SEQ ID NO: 1 or a complementary sequence thereof, and/or
the differentially methylated region shown in item (2) has a base sequence as set forth in SEQ ID NO: 2 or a complementary sequence thereof.

3. The differentially methylated region according to any one of claims 1 to 2, which is used for detecting a colorectal cancer or a colorectal cancer precancerous lesion, or for assessing the prognostic risk of a colorectal cancer patient.

4. The differentially methylated region according to any one of claims 1 to 3, which has undergone the following treatment:
a bisulfite treatment, a methylation sensitive endonuclease treatment, a methylation restriction endonuclease treatment or a methylation modification enzyme treatment.

5. A kit, comprising a reagent for detecting the differentially methylated region according to any one of claims 1 to 4 or a reagent for detecting a methylation site in the differentially methylated region.

6. The kit according to claim 5, wherein the reagent comprises:
1) a primer and a probe for detecting the differentially methylated region shown in item (1) or a methylation site in the differentially methylated region;
2) a primer and a probe for detecting the differentially methylated region shown in item (2) or a methylation site in the differentially methylated region;
preferably, the reagent further comprises:
3) a primer and a probe for an internal reference gene.

7. The kit according to claim 6, wherein:
in 1), the sequence of the primer is selected from SEQ ID NOs: 3 to 79, and the sequence of the probe is selected from SEQ ID NOs: 160 to 188; and in 2), the sequence of the primer is selected from SEQ ID NOs: 80 to 159, and the sequence of the probe is selected from SEQ ID NOs: 189 to 209;
preferably:
in 1), the sequence of the primer is selected from SEQ ID NO: 30 and SEQ ID NO: 32, and the sequence of the probe is selected from SEQ ID NO: 170; and in 2), the sequence of the primer is selected from SEQ ID NOs: 108 to 109, and the sequence of the probe is selected from SEQ ID NO: 197;
in 1), the sequence of the primer is selected from SEQ ID NOs: 25 to 26, and the sequence of the probe is selected from SEQ ID NO: 164; and in 2), the sequence of the primer is selected from SEQ ID NOs: 144 to 145, and the sequence of the probe is selected from SEQ ID NO: 204;
in 1), the sequence of the primer is selected from SEQ ID NOs: 26 to 27, the sequence of the probe is selected from SEQ ID NO: 168; and in 2), the sequence of the primer is selected from SEQ ID NOs: 154 to 155, the sequence of the probe is selected from SEQ ID NO: 206;
in 1), the sequence of the primer is selected from SEQ ID NOs: 33 to 34, the sequence of the probe is selected from SEQ ID NO: 171; and in 2), the sequence of the primer is selected from SEQ ID NOs: 156 to 157, the sequence of the probe is selected from SEQ ID NO: 209;
in 1), the sequence of the primer is selected from SEQ ID NOs: 56 to 57, the sequence of the probe is selected from SEQ ID NO: 184; and in 2), the sequence of the primer is selected from SEQ ID NOs: 158 to 159, the sequence of the probe is selected from SEQ ID NO: 203;
in 1), the sequence of the primer is selected from SEQ ID NOs: 61 to 62, the sequence of the probe is selected from SEQ ID NO: 183; and in 2), the sequence of the primer is selected from SEQ ID NOs: 108 to 109, the sequence of the probe is selected from SEQ ID NO: 191;
in 1), the sequence of the primer is selected from SEQ ID NOs: 33 to 34, the sequence of the probe is selected from SEQ ID NO: 171; and in 2), the sequence of the primer is selected from SEQ ID NOs: 108 to 109, the sequence of the probe is selected from SEQ ID NO: 191;
or,
in 1), the sequence of the primer is selected from SEQ ID NOs: 25 to 26, the sequence of the probe is selected from SEQ ID NO: 164; and in 2), the sequence of the primer is selected from SEQ ID NOs: 158 to 159, the sequence of the probe is selected from SEQ ID NO: 203;
preferably, in 3), the sequence of the primer of the internal reference gene is set forth in SEQ ID NOs: 210 to 211, and the sequence of the probe of the internal reference gene is set forth in SEQ ID NO: 212.

8. The kit according to any one of claims 5 to 7, which further comprises one or more selected from the following:
a stool sample sampler, a nucleic acid extraction reagent, a methylation detection sample pretreatment reagent, a PCR reagent, and a stool occult blood detection reagent;
preferably, the stool occult blood detection reagent is a stool immunochemical detection reagent, a guaiac detection reagent, a tetramethylbenzidine detection reagent, or an ELISA double antibody sandwich detection reagent;
preferably, the stool immunochemical detection reagent is an immunocolloidal gold stool occult blood detection reagent.

9. The kit according to claim 8, wherein the methylation detection sample pretreatment reagent is a bisulfite, a methylation-sensitive endonuclease, a methylation restriction endonuclease, or a methylation modification enzyme.

10. Use of the differentially methylated region according to any one of claims 1 to 4, or a reagent for detecting the differentially methylated region according to any one of claims 1 to 4 or a methylation site in the differentially methylated region, in the manufacture of a medicament for detecting a colorectal cancer or a colorectal cancer precancerous lesion, or for assessing the prognostic risk of a colorectal cancer patient.

11. The use according to claim 10, wherein the reagent is a primer and a probe for detecting the differentially methylated region according to any one of claims 1 to 4 or a methylation site in the differentially methylated region.

12. A method for detecting a colorectal cancer or a colorectal cancer precancerous lesion or assessing the prognostic risk of a colorectal cancer patient, comprising the following steps:
detecting the content of the differentially methylated region according to any one of claims 1 to 4 or the content of a fragment thereof; wherein the fragment comprises one or more methylation sites in the differentially methylated region.

13. The method according to claim 12, wherein the content of each differentially methylated region according to any one of claims 1 to 4 or a fragment thereof is detected by a fluorescent quantitative PCR method.

14. The method according to claim 13, wherein,
the content of human DNA in a sample is evaluated by an internal reference gene Ct value, when the internal reference gene Ct value is >37, the sample human DNA content is judged to be too low, and the current detection fails, when the internal reference gene Ct value is ≤37, the sample is evaluated to be qualified and can be analyzed later.

15. The method according to claim 14, wherein, for the sample that has passed the evaluation:
when the Ct value of each differentially methylated region is greater than 38 or no Ct value is detected, it is judged to have negative detection result;
when the Ct value of each differentially methylated region is less than or equal to 38, the difference △Ct value between each differentially methylated region and the internal reference gene Ct value is further calculated.

16. The method according to any one of claims 13 to 15, wherein:
if the △Ct of item (1) or item (2) is less than or equal to a cutoff value, it is judged to have a high risk of colorectal cancer, otherwise it is judged to have a low risk;
wherein △Ct = (Ct value of the differentially methylated region or Ct value of the amplified fragment) - Ct value of the reference gene;
wherein the amplified fragment comprises one or more methylation sites in the differentially methylated region.

17. The method according to any one of claims 12 to 16, which further comprises a step of performing an evaluation in combination with a fecal occult blood detection result.

18. The method according to any one of claims 12 to 17, wherein the sample is a human tissue sample, a blood sample, a cell sample, a secretion sample or an excrement sample such as a fecal sample.

19. A model for diagnosing or evaluating a colorectal cancer, which uses a logical function g(y)=1/(1+e^{-y}) for calculation, wherein:
g(y) represents a risk index, when the risk index is higher than a preset value, it is judged to be positive or a positive candidate;
e represents the natural constant;
y=(θ0+θ1*X1+θ2*X2+...+θn*Xn), θ0 represents a constant term, θi (i=1, 2, ..., n) represents a coefficient to be determined, Xi (i=1, 2, ..., n) represents a detection result of the differential methylation region according to any one of claims 1 to 4 and a detection result of fecal occult blood.

20. A system for diagnosing or evaluating a colorectal cancer, comprising:
1) a DMR detection unit: which is configured to obtain content data of the differentially methylated region according to any one of claims 1 to 4 or a fragment thereof; wherein the fragment comprises one or more methylation sites in the differentially methylated region;
2) a fecal occult blood detection unit: which is configured to obtain fecal occult blood detection result data;
3) a data analysis unit: which is configured to input the data obtained by the DMR detection unit and the fecal occult blood detection unit into the model for diagnosing or evaluating colorectal cancer according to claim 19 for analysis;
4) a risk assessment unit: which is configured to output a risk index or judgment result of an individual sample.

21. Use of the differentially methylated region according to any one of claims 1 to 4 in the manufacture of a product for detecting a colorectal cancer or a colorectal precancerous lesion.
